# FASCICULE DE BREVET EUROPEEN

(11) **EP 1 878 790 B1**
(45) Date de publication et mention de la délivrance du brevet: **23.08.2017**
(21) Numéro de dépôt: 07111537.2
(22) Date de dépôt: 02.07.2007
(51) Int. Cl.: C12N 5/071

(54) **EQUIVALENT D`ÉPIDERME CAPABLE DE SE PIGMENTER OBTENU À PARTIR DE CELLULES DE LA MATRICE, PROCÉDÉ DE PRÉPARATION ET UTILISATION**
EPIDERM EQUIVALENT, CAPABLE OF PIGMENTING, OBTAINED FROM MATRIX CELLS, METHOD OF PREPARATION AND USE
EPIDERMIS EQUIVALENT, FÄHIG SICH ZU PIGMENTIEREN, ERHÄLTLICH AUS MATRIXZELLEN, VERFAHREN ZUR HERSTELLUNG UND VERWENDUNG

(30) Priorité: 13.07.2006 FR 0652984
(43) Date de publication de la demande: 16.01.2008
(73) Titulaire: L'Oréal, 75008 Paris (FR)
(72) Inventeur: Bakkar, Khalid, 92400 Courbevoie (FR); Asselineau, Daniel, 91440 Bures sur Yvette (FR)
(74) Mandataire: Pillet, Anne-Helene

(56) Documents cités:
- WO-A-2005/007835
- FR-A1- 2 665 175
- US-A1- 2005 089 512

## Description

La présente invention se rapporte à un épiderme équivalent capable de se pigmenter comprenant des cellules issues de la différentiation des cellules de la matrice, cellules localisées dans le bulbe pileux et forment un petit amas cellulaire autour de la papille dermique ainsi que son procédé de préparation.
La présente invention se rapporte également à une peau reconstruite capable de se pigmenter et comprenant éventuellement des follicules pileux, comprenant ledit équivalent d'épiderme, son procédé de préparation et son utilisation pour évaluer l'effet de produits topiques cosmétique, pharmaceutique ou dermatologique.
La peau reconstruite selon l'invention pourra encore être utilisée pour la préparation de greffons destinés à être transplantés sur des mammifères, plus particulièrement sur des patients humains tels que des grands brûlés.

On cherche à mettre au point, depuis de nombreuses années, des modèles de peau reconstruite qui permettent, d'une part, d'effectuer les études nécessaires à la meilleure compréhension du rôle de la peau tant dans le domaine mécanique que dans le domaine physiologique, et d'autre part, qui constituent des tests prédictifs de l'activité d'actifs cosmétiques et/ou pharmaceutiques ou encore des effets secondaires d'ingrédients topiques.

Ainsi, des modèles plus ou moins proches de la peau humaine ont pu être mis au point. On peut citer par exemple les modèles décrits dans les documents suivants : EP 0 285 471, EP 0 285 474, EP 0 418 035, WO-A-90/02796, WO-A-91/16010, EP 0 197 090, EP 0 020 753, FR 2 665 175, FR 2 689 904.

De manière générale, les modèles de peau reconstruite décrits dans ces documents sont préparés à partir de fibroblastes de la papille dermique et/ou de cellules prélevées dans la gaine épithéliale externe (encore appelée ORS pour Outer Root Sheath), en raison de la facilité d'obtention de ces cellules. En effet, ces cellules sont prélevées lorsqu'on arrache un cheveu et sont capables de fabriquer un épiderme lorsqu'elles sont déposées sur un support, souvent un équivalent de derme, et cultivées dans un milieu de culture adapté.

Lorsque les kératinocytes proviennent de l'ORS, les quelques mélanocytes qui peuvent être présents dans ces modèles d'épiderme sont peu nombreux et ne permettent pas d'obtenir un modèle de peau pigmenté satisfaisant (Lenoir MC, Bernard BA, Pautrat G, Darmon M, Shroot B. Outer root sheath cells of human hair follicle are able to regenerate a fully differentiated epidermis in vitro.Dev Biol. 1988 Dec;130(2):610-20 et Limat A, Breitkreutz D, Hunziker T, Boillat C, Wiesmann U, Klein E, Noser F, Fusenig NE. Restoration of the epidermal phenôtype by follicular outer root sheath cells in recombinant culture with dermal fibroblasts.Exp Cell Res. 1991 Jun;194(2):218-27).

Dans le but d'obtenir des modèles de peaux pigmentées, il est connu d'ajouter à une culture de kératinocytes des mélanocytes épidermiques disponibles commercialement (FR 2 665 175). Cependant, la nature de la pigmentation obtenue avec un tel modèle ne reproduit pas de façon satisfaisante la pigmentation naturelle.

Les cellules de la matrice, qui sont localisées dans le bulbe pileux et forment un petit amas cellulaires autour de la papille dermique, sont majoritairement constituées de précurseurs de kératinocytes qui constituent une assise germinative et qui prolifèrent rapidement pour se différencier en formant la tige pilaire, jouant ainsi un rôle essentiel dans le cycle pilaire. Du début de la phase anagène jusqu'à la fin de celle-ci, ces cellules matricielles vont proliférer jusqu'à la phase catagène puis disparaître en phase télogène. (Ebling FJ. The biology of hair.Dermatol Clin. 1987 Jul;5(3):467-81. Review; Saitoh M, Uzuka M, Sakamoto M. Human hair cycle. J Invest Dermatol. 1970 Jan;54(1):65-81). La matrice comprend également des mélanocytes folliculaires qui sont responsables de la pigmentation du cheveu.

La prolifération et la différenciation de ces cellules de la matrice, cellules localisées dans le bulbe pileux et forment un petit amas cellulaire autour de la papille dermique, sont contrôlées par la papille dermique (Botchkarev VA, Kishimoto J. Molecular control of epithelial-mesenchymal interactions during hair follicle cycling. J Invest Dermatol Symp Proc. 2003 Jun;8(1):46-55. Review).

De façon surprenante, la demanderesse a mis en évidence que la culture sur un derme artificiel des cellules de la matrice cellules localisées dans le bulbe pileux et forment un petit amas cellulaire autour de la papille dermique pouvait conduire à un épiderme complet, comprenant notamment kératinocytes et mélanocytes.

Bien que les mélanocytes folliculaires présents dans la matrice soient normalement destinés à colorer le cheveu, ils s'avèrent également capables de croitre dans un environnement épidermique et aussi fonctionnels pour pigmenter des épidermes produits *in vitro* selon le procédé de la présente invention, mais aussi *in vivo* après greffe. Ainsi, la demanderesse a mis au point un nouveau procédé de préparation d'un modèle de peau capable de se pigmenter à partir exclusivement de cellules du cheveu, les cellules de la matrice, cellules localisées dans le bulbe pileux et forment un petit amas cellulaire autour de la papille dermique.

Ce procédé se révèle avantageux par le fait qu'il permet d'obtenir un modèle de peau comprenant un épiderme complet en utilisant un seul tissu, la matrice, cellules localisées dans le bulbe pileux et forment un petit amas cellulaire autour de la papille dermique qui apporte tous les types cellulaires de l'épiderme, de plus, les cellules sont compatibles entre elles, dans un état peu différencié qui leur permet de se multiplier en formant un tissu épidermique qui reproduit les caractéristiques d'un épiderme vivant.
Ce procédé de préparation d'équivalent d'épiderme ne nécessite pas de prétraitement visant à enrichir le tissu en un type cellulaire donné.
Les cellules de la matrice peuvent être utilisées quelle que soit la phase dans laquelle est le cheveu : anagène, catagène ou télogène.
Enfin, ce procédé nécessite un nombre très faible de cellules (on ensemence environ 1000 cellules) par rapport aux techniques déjà décrites dans la littérature utilisant des kératinocytes de l'ORS (qui, elles, nécessitent l'ensemencement d'environ 33000 cellules) et la cinétique de colonisation des cellules de la matrice étant élevée, la préparation des épidermes reconstruits en est accélérée.

Une difficulté à surmonter réside dans l'obtention des cellules de la matrice, cellules localisées dans le bulbe pileux et forment un petit amas cellulaire autour de la papille dermique, de qualité et en quantité suffisante.
Dans les conditions normales, lorsqu'un cheveu tombe ou après arrachage, le compartiment papille dermique-cellules de la matrice reste dans le derme du scalp. Ce compartiment initiera le développement d'un nouveau cycle pilaire et redonnera un nouveau follicule. Ainsi, l'arrachage d'un cheveu ne permet pas d'obtenir ces cellules matricielles, il faudra donc réaliser une biopsie de cuir chevelu, puis isoler ces cellules par microdissection.

En plus d'être difficiles à isoler, les cellules de la matrice, cellules localisées dans le bulbe pileux et forment un petit amas cellulaire autour de la papille dermique, sont également difficiles à cultiver au moins pour les raisons suivantes :
a) leur nombre est particulièrement faible ;
b) elles se dissocient facilement donc il est difficile de les repérer lors de la microdissection ;
c) elles n'adhèrent pas sur un support plastique ;
d) elles sont difficiles à amplifier car elles ont tendance à former des petites colonies et non une primo culture à confluence nécessaire à la préparation d'un épiderme équivalent.

Certains auteurs ont proposé de cultiver ces cellules en co-culture avec les fibroblastes de la papille. L'inconvénient de cette méthode est que la culture obtenue n'est pas homogène, les cellules de la matrice sont contaminées avec les fibroblastes dermiques de la papille utilisés en co-culture (Reynolds AJ, Lawrence CM, Jahoda CA Human hair follicle germinative epidermal cell culture. J Invest Dermatol.1993 Oct;101(4):634-8).

Luo *et al.* ont proposé une technique de microdissection puis de culture des cellules de la matrice sur support plastique (Luo et al : Methods for culturing hair follicle epithelial matrix cells ; Reg. number: H1610; Nov.5, 1996). Cependant cette technique de microdissection conduit à un très faible rendement en cellules de là matrice et la culture de ces cellules permet uniquement d'obtenir une monocouche de cellules non différenciées qui ne peut servir de modèle de peau complet.

La demanderesse a isolé les cellules de la matrice, cellules localisées dans le bulbe pileux et forment un petit amas cellulaire autour de la papille dermique, et mis au point un procédé de mise en culture de ces cellules sur un derme équivalent, ces cellules prolifèrent rapidement et se différencient pour former un épiderme complet.

De façon préférentielle, le tissu matriciel est obtenu par une nouvelle technique de microdissection qui préserve la quantité et l'intégrité des cellules, elles ne sont pas séparées les unes des autres. Cette microdissection permet d'isoler et de conserver la totalité des cellules de la matrice sur un support de derme équivalent.

Avec cette technique de dissection, les cellules de la matrice adhèrent plus facilement sur l'équivalent du derme, prolifèrent uniformément et se différencient. L'équivalent d'épiderme obtenu pourra en outre comprendre des types cellulaires autres que les kératinocytes tels que les mélanocytes.

Les techniques connues de préparation d'épidermes capables de se pigmenter utilisent des kératinocytes de l'ORS auxquels des mélanocytes sont ajoutés après une pré-culture *in vitro,* cependant, une conséquence de cette pré-culture est que la différenciation des cellules se fait en dehors du contexte épidermique, les mélanocytes ne développent pas une quantité de dendrites comparables aux mélanocytes observés *in vivo.* Leur introduction postérieure dans le modèle d'épiderme ne garantit pas une répartition homogène des mélanocytes au niveau de la couche basale de kératinocytes, ni une fonctionnalité optimale de ces mélanocytes. Enfin, les mélanocytes obtenus sont généralement bipolaires (deux dendrites) et ont donc une faible capacité de transmission des grains de mélanines aux kératinocytes.

Ainsi selon un premier de ses objets, la présente invention se rapporte à l'utilisation de cellules de la matrice pour la préparation d'un équivalent d'épiderme pigmenté.

On entend par « cellules de la matrice », les cellules localisées dans le bulbe pileux et forment un petit amas cellulaire autour de la papille dermique (Ebling FJ. The biology of hair.Dermatol Clin. 1987 Jul;5(3):467-81. Review; Saitoh M, Uzuka M, Sakamoto M. Human hair cycle. J Invest Dermatol. 1970 Jan;54(1):65-81). Ces cellules pourront être échantillonnées, amplifiées et conservées en banques de tissus.
Pour préserver l'intégrité du tissu matriciel, ces cellules pourront être prélevées selon le procédé qui suit : des follicules pileux sont placés dans une boite de Pétri comprenant un milieu de culture minimum additionné de 2% d'antibiotique, des acides aminés essentiels. L'épithélium du bulbe est séparé de la papille dermique et de la gaine conjonctive puis la région bulbaire est coupée au sommet de la papille dermique. Le fragment obtenu est ensuite placé sur un derme équivalent à l'intérieur d'un anneau stérile puis les cellules de la matrice sont mises en culture dans un milieu détaillé dans le procédé de préparation de l'épiderme ci-après et dans l'exemple I.

Les cellules de la matrice apportent toutes les cellules nécessaires à la préparation d'un épiderme capable de se pigmenter, ainsi, l'utilisation de cellules de la matrice pour la préparation d'un équivalent d'épiderme ne nécessite aucun autre type de cellule et pourra se faire à l'exclusion de tout apport de cellules exogènes.

Selon un second objet, la présente invention se rapporte à un procédé de préparation d'un équivalent d'épiderme comprenant au moins une étape de mise en culture de cellules de la matrice sur un équivalent de derme.

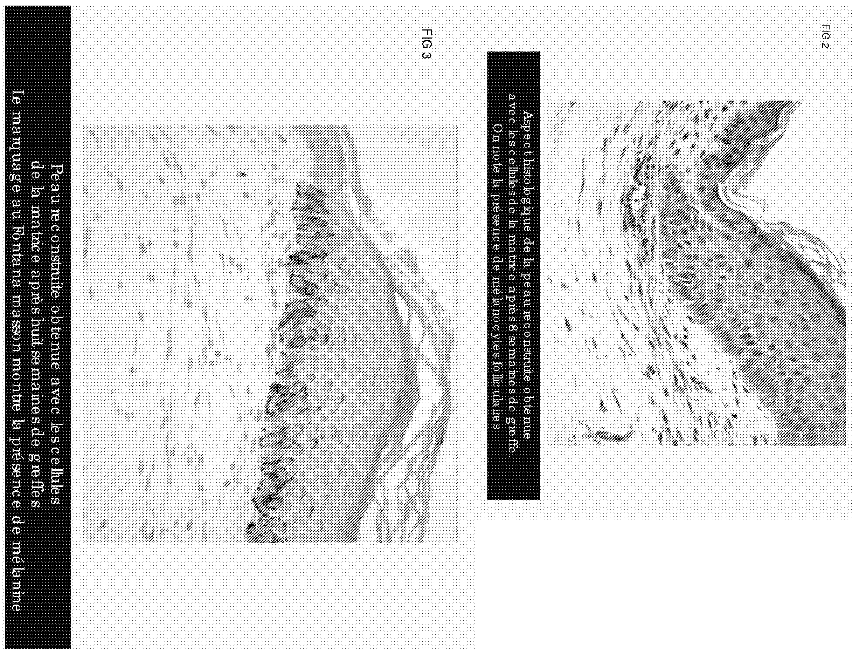

L'« équivalent d'épiderme » -encore désigné par modèle d'épiderme- obtenu selon le procédé de l'invention est un tissu complet reproduisant les caractéristiques d'un épiderme *in vivo,* à savoir, un épithélium multicouche stratifié kératinisé, comprenant au minimum des kératinocytes, avec des cellules basales en contact avec un derme équivalent et dont les strates supérieures témoignent de la différentiation terminale reproduisant un *stratum granulosum* et un *stratum corneum* histologiquement normaux. Cet équivalent d'épiderme comprend également des mélanocytes fonctionnels, c'est-à-dire capables de transférer des mélanosomes (grains de mélanine) aux kératinocytes, l'observation histologique de l'épiderme montre une organisation des mélanocytes au niveau de la couche basale reproduisant ainsi fidèlement la structure normale de la peau.
Une caractéristique de ce modèle d'épiderme est un nombre élevé de mélanocytes comportant de nombreuses dendrites (trois ou plus), englobant régulièrement les kératinocytes basaux, ces caractéristiques apparaissent très clairement lors de l'observation d'une coupe transversale de modèle d'épiderme (voir notamment la figure 3 où il est visible que les kératinocytes de la couche basale sont entourés d'une couche épaisse et sombre correspondant aux mélanocytes).
Ces caractéristiques permettent en particulier de distinguer un modèle d'épiderme obtenu selon le procédé de l'invention par rapport aux procédés de l'état de la technique. En effet, les épidermes préparés selon les procédés classiques (ajout ultérieur de mélanocytes) comportent un faible nombre de mélanocytes, positionnés entre des kératinocytes basaux avec deux dendrites.

De préférence, l'équivalent d'épiderme comprend également des cellules de Langerhans et/ou des cellules de Merkel.

De plus, cet épiderme est capable de se pigmenter de façon uniforme.

En effet, il apparaît que le modèle d'épiderme selon invention présente une pigmentation homogène avec des grains de mélanine régulièrement positionnés dans le pôle supérieur des kératinocytes ainsi que dans le *stratum corneum.* Cette répartition est avantageuse par rapport aux modèles d'épiderme de l'art antérieur qui eux présentent moins de grains de mélanines distribués irrégulièrement dans l'épiderme.

Plus spécifiquement, le procédé selon l'invention est caractérisé en ce qu'il comprend :
a- l'ensemencement de cellules de la matrice, cellules localisées dans le bulbe pileux et
forment un petit amas cellulaire autour de la papille dermique, sur un équivalent de derme reposant sur un support immergé dans un milieu de culture ;
b- la multiplication des cellules de la matrice, cellules localisées dans le bulbe pileux et forment un petit amas cellulaire autour de la papille dermique, à la surface dudit équivalent de derme ;
c- l'élévation dudit support de telle sorte que le milieu de culture ne recouvre pas la face supérieure de l'équivalent d'épiderme en cours d'élaboration.

En particulier, le support peut être une grille-support.

L'« équivalent de derme », ou support dermique, utilisé selon l'invention peut être l'un quelconque de ceux décrits dans l'art antérieur (notamment ceux décrits dans le FR 2 665 175). A titre d'exemple, on peut citer comme support les lattices contractées collagène/fibroblastes, le derme préalablement désépidermisé (Prunieras et collaborateurs, Ann. Chir. Plast., 1979, 24, n°4, 357-362), un support inerte choisi parmi le groupe consistant en une membrane synthétique semi-perméable (notamment ceux décrits dans le FR 2 833 271), les membranes artificielles comme par exemple les filtres de marque Millipore, les substituts sous-cutanés à base de collagène, ou tout autre support exposable à l'air compatible avec la viabilité et l'adhésion cellulaire.

De préférence, le derme équivalent consistera en une lattice de collagène dans laquelle se répartissent des fibroblastes dermiques qui sont dans un état de différentiation équivalent à celui observé *in vivo.*
Ce type d'équivalent de derme peut être préparé selon différents procédés, par exemple tel que décrit dans l'EP 0 418 035, le WO 00/29553 ou encore l'EP 0 285 471.
Ce procédé pourra comprendre les étapes suivantes de culture :
a) de cellules contractiles récoltées, par exemple, à partir de cultures monocouches réalisées sur un milieu nutritif ensemencé par des fragments de tissu animal ou humain avec
b) un milieu nutritif additionné de composants de la matrice extracellulaire du derme, ledit mélange formant un gel qui se contracte en expulsant le milieu nutritif pour former l'équivalent de derme.

On peut utiliser comme cellule contractile, des fibroblastes ; on utilise, avantageusement, comme cellules contractiles, des fibroblastes dermiques obtenus à partir de donneurs humains sains et récoltées, à partir des cultures monocouches, par trypsinisation ménagée. Comme expliqué ci-après, le choix des cellules contractiles conditionnera la différentiation des cellules de la matrice, leur choix devra donc se faire en fonction de la qualité de l'épiderme équivalent désiré.

Les cellules de la matrice constituent un réservoir de cellules pluripotentes capables, en fonction du contexte cellulaire créé par l'équivalent de derme, de former de la peau ou du cheveu, car le fibroblaste de l'équivalent de derme peut diriger le programme de différentiation du kératinocyte.
Ainsi, si l'équivalent de derme sur lequel reposent les cellules de la matrice contient majoritairement des fibroblastes de peau inferfolliculaire, du fait de l'interaction entre les fibroblastes et les kératinocytes de la matrice, les kératinocytes de la matrice formeront un équivalent d'épiderme interfolliculaire.
Le fibroblaste de peau pourra être un fibroblaste papillaire ou réticulaire ou l'addition des deux.
Pour la préparation d'un modèle d'épiderme capable de se pigmenter, on pourra utiliser un fibroblaste interfolliculaire.

La mise en oeuvre du procédé de préparation selon l'invention consiste à ensemencer l'équivalent de derme -ou substrat dermique- avec les cellules de la matrice.
Des matrices entières sont déposées sur le derme équivalent. Le nombre de matrices déposées dépend de la surface de l'équivalent du derme. Par exemple lorsque les équivalents dermiques sont réalisés dans une boîte de 60 mm, ceux-ci, après contraction, ont une surface de 2 cm² environ et sont ensemencés avec environ 10 matrices.

On maintient des conditions permettant la multiplication des cellules de la matrice à la surface dudit substrat, le développement de cette culture de cellules de la matrice étant favorisé par l'utilisation d'au moins un milieu nutritif minimum, de préférence un milieu 3F tel que décrit dans l'exemple 1 au contact desdites cellules de la matrice.

Avantageusement, après l'ensemencement des cellules de la matrice sur le substrat, on maintient, de préférence entre 5 à 6 jours, le substrat implanté en immersion dans ce milieu nutritif qui recouvre les cellules de la matrice.

Ensuite, on place l'équivalent de derme en contact avec l'air, pour cela, on le place sur une grille-support surélevée par rapport au fond du réceptacle et on ajuste le niveau du milieu nutritif, pour que la grille-support soit juste recouverte mais que le milieu nutritif ne recouvre pas la face supérieure de l'équivalent de peau en cours d'élaboration.

Selon une variante, le procédé selon l'invention pourra en outre comprendre une étape supplémentaire visant à induire la pigmentation de l'équivalent d'épiderme. Il pourra s'agir d'une stimulation par un rayonnement UV.

L'équivalent de peau selon l'invention ainsi constitué est bien différencié, bien organisé. Il est aussi globalement homogène.

On entend par « équivalent de peau », aussi désigné peau reconstruite ci-après, l'ensemble équivalent d'épiderme reposant sur un derme équivalent.

L'équivalent d'épiderme ainsi préparé comprend au moins des kératinocytes et des mélanocytes.
L'équivalent d'épiderme peut aussi comprendre des cellules de Langerhans et/ou des cellules de Merkel, ces cellules pourront notamment être ajoutées sur le support lors de l'ensemencement des cellules de la matrice.

Selon une variante du procédé selon l'invention, il est possible de préparer un équivalent de peau capable de reconstituer un ou plusieurs follicules pileux en utilisant un équivalent de derme comportant des fibroblastes de la papille dermique et/ou des fibroblastes de la gaine conjonctive et/ou des papilles dermiques entières.

S'il est fait le choix d'inclure dans l'équivalent de derme des fibroblastes du cheveu tels que les fibroblastes de la papille ou les fibroblastes de la gaine conjonctive ou la papille entière ou la gaine conjonctive ou des fractions de la gaine conjonctive (Reynolds AJ, Lawrence CM, Jahoda CA Human hair follicle germinative epidermal cell culture. J Invest Dermatol.1993 Oct;101(4):634-8), en raison de l'interaction entre ces fibroblastes et les cellules de la matrice, les kératinocytes reproduiront la morphogenèse du poil ou du cheveu (tige pilaire).

Un autre objet de la présente invention se rapporte à un équivalent d'épiderme capable de se pigmenter comportant des mélanocytes comportant trois dendrites ou plus entourant les kératinocytes basaux. caractérisé en ce qu'il est constitué de cellules issues de la prolifération et de la différentiation des cellules de la matrice comprenant au moins des kératinocytes et des mélanocytes.

Il s'agit plus spécifiquement d'un équivalent d'épiderme préparé selon le procédé de l'invention, l'équivalent d'épiderme présente la particularité de pouvoir se pigmenter uniformément et de façon homogène reproduisant la pigmentation d'une peau saine à la fois *in vitro* et *in vivo* après greffe.

La pigmentation du modèle de peau ou d'épiderme (synthèse de mélanine par les mélanocytes) peut être quantifiée par toute méthode connue, notamment, par une coloration dite de Fontana Masson.

A titre d'exemple et sans caractère limitatif, la pigmentation peut s'observer et/ou se quantifier par :
- mesure du nombre de grains de mélanine dans un certain nombre de coupes verticales colorées par analyse d'images assistée par ordinateur ;
- mesure du contenu en mélanine des homogénats de culture par des techniques biochimiques ;
- mesure de l'activité de la tyrosinase épidermique contenue dans les cultures par techniques biochimiques ;
- mesure du nombre de mélanocytes présents et de leur localisation dans les cultures par coloration à la DOPA, par des techniques d'immunofluorescences indirecte ou de microscopie électronique ;
- mesure instrumentale de la couleur par colorimétrie qui tient compte de l'équilibre variable entre les différents pigments et leurs états physico-chimiques ;
- toute autre technique permettant d'évaluer précisément l'intensité de la pigmentation obtenue.

La Demanderesse a également pu mettre en évidence qu'après une greffe, l'équivalent d'épiderme présentait avantageusement cette capacité de se pigmenter. De plus, la pigmentation obtenue est homogène, uniforme et identique à celle de la peau d'origine.

Quelque soit la variante de procédé retenue pour la préparation de la peau équivalente, l'équivalent d'épiderme et/ou de peau est un modèle tridimensionnel utile comme test alternatif pour tous les tests qui nécessiteraient des expérimentations animales, par exemple, les études de libération d'actifs, de leur pénétration et/ou absorption et/ou biodisponibilité cutanée, les études de tolérance, de compatibilité, d'efficacité d'actifs et/ou d'ingrédients cosmétiques, pharmaceutiques ou dermatologiques.

L'équivalent d'épiderme et/ou de peau selon l'invention peut aussi être utilisé dans des procédés automatisés de criblages de produits cosmétiques, pharmaceutiques ou dermatologiques pour identifier de nouveaux actifs.

L'équivalent de peau selon l'invention permet également l'étude physiologique du mécanisme de pigmentation de la peau ainsi que de la pousse des cheveux et/ou poils.

L'équivalent d'épiderme selon l'invention reproduisant la pigmentation naturelle de la peau, il est particulièrement avantageux pour tester des actifs dépigmentants, des actifs pigmentants, des filtres solaires.

Les applications peuvent se révéler particulièrement utiles pour le photovieillissement ou le vieillissement notamment lorsqu'on recherche des produits capables d'éclaircir des « taches de vieillesse », ou lentigos, ou plus généralement éclaircir le teint de la peau.

Les équivalents d'épiderme comportant des follicules pileux permettront de réaliser notamment des cinétiques de pousse des poils ou cheveux et donc toute étude nécessitant de nombreux cheveux vivants et aussi complets que possibles dans un contexte *in vivo* tel que l'étude du cycle du cheveu et des facteurs capables d'influencer ce cycle allant jusqu'à l'étude d'actifs favorisant la croissance du cheveu, d'actifs permettant de lutter contre la chute des cheveux ou encore d'actifs ralentissant la pousse des poils.
De par la présence de mélanocytes actifs, ce modèle permet également l'étude d'actifs pouvant influencer la couleur des cheveux et/ou des poils, notamment par l'induction ou l'inhibition de production de mélanine.
Enfin, ce modèle permet également d'étudier l'effet d'actifs destinés à modifier la structure des poils et/ou des cheveux.

Les procédés de criblage de produit en vue d'identifier de nouveaux actifs comprennent une étape (a) de mise en contact dudit produit à tester avec un épiderme équivalent selon l'invention puis une étape (b) de lecture de l'effet du produit sur l'équivalent d'épiderme. Selon une variante du procédé de criblage selon l'invention, on utilisera un modèle de peau complet.
De préférence, pour la mise en oeuvre de l'étape (a), le produit à tester sera appliqué de façon topique, par exemple, formulé dans des formulations topiques classiques ou bien introduit dans le milieu de culture.
Selon l'effet recherché, l'étape (b) pourra consister, de façon non exhaustive, à comparer avec un équivalent d'épiderme selon l'invention n'ayant pas été en contact avec le produit à tester (contrôle) et/ou à observer une modification de l'intensité de la pigmentation ou de la dépigmentation de l'épiderme équivalent, éventuellement par marquage et quantification de la mélanine (par exemple par coloration selon la méthode de Fontana Masson), une accélération ou une diminution de la pousse de la tige pilaire, éventuellement un effet sur la production des kératines du cheveu.
L'étape (b) pourra également être réalisée par l'analyse de marqueurs épidermiques et/ou dermiques, tels que des protéines de structure, notamment, des protéines de différentiation épidermique et macromoléculaires de la matrice dermique.

Les paramètres mesurés sur les épidermes ou peaux équivalents mis en contact avec le produit à tester seront comparés à ceux mesurés sur des épidermes ou peaux équivalents témoins cultivés dans les mêmes conditions mais qui n'auront pas reçu le produit à tester.

Lorsque le modèle d'épiderme ou de peau comprend des cellules du système immunitaire, les cellules de Langerhans, les tests de criblage pourront également être destinés à identifier des produits susceptibles d'induire des irritations ou des réactions allergiques.

Pour cela, les paramètres observés à l'étape (b) du procédé de criblage seront choisis parmi :
- la cytotoxicité ;
- la libération de médiateurs de l'inflammation ;
- les dommages cellulaires révélés par histologie ou par la libération de lactate dehydrogénase (marqueur de l'intégrité membranaire des kétatinocytes) (Roguet et al J Tox in vitro 6:303 (1992) et Ponec M dans In Vitro Toxicology. Eds Rougier, Maibach et Golberg p 107, 1994) ;
- la modification de la synthèse et de la composition des lipides de la peau, particulièrement les céramides et les phospholipides (JID 86,598 (1986)) ;
- la stratification des cellules épithéliales comme marqueur de leur différenciation (M Prunieras & R Roguet Toxicologie cellulaire in vitro methodes & applications Eds M adolphe, A Guillouzo et F Marano eds INSERM 1995 p 191-236)(DUFFY PA, FLINT OP: In vitro dermal irritancy test. In CK Atterwill and CE Steele (Eds): In vitro methods in Toxicology Cambridge Univ. Press Cambridge 1987, pp279-297)(Use of skin cell culture for in vitro assessment of corrosion and cutaneous irritancy, Roguet, Cell Biology and Toxicology, 1999:15, 63-75).

L'équivalent d'épiderme trouvera également des applications pour la préparation de greffons destinés à être transplantés sur des patients présentant un désordre cutané tel qu'une brûlure, un défaut de cicatrisation, un ulcère cutané, le psoriasis, un désordre pigmentaire tel que le vitiligo, la canitie.
L'application concernera aussi toute pathologie se traduisant par des anomalies de pigmentation graves pour lesquelles une correction par greffe est envisageable, par exemple hypomelanoses ou hypermelanoses congénitales ou acquises (Na GY, Seo SK, Choi SK, Single hair grafting for the treatment of vitiligo. J Am Acad Dermatol. 1998 Apr; 38(4):580-4.), des causes accidentelles, par exemple une peau ayant été brûlée sur une grande surface du corps ou du visage (ex: grands brûlés) ; des causes chirurgicales, par exemple une peau ayant subi une excision pour le traitement d'un nevus (Kumagai et al., Ann Plast Surg. 39: 483-488, 1997), ou d'un tatouage (Kumagai et al., An Plast Surg. 33 : 385-391, 1994).
L'application concerne encore des désordres liés à la dénaturation de la matrice.

L'équivalent de peau selon l'invention permet également de réaliser des autogreffes lorsqu'il est préparé à partir de fragments de scalp de cuir chevelu après extraction par microdissection des matrices des cheveux conduisant à la formation rapide *in vivo* d'un épiderme uniformément pigmenté. Ces autogreffes peuvent concerner les ulcères mais aussi les brûlures graves (Limat A, French LE, Blal L, Saurat JH, Hunziker T, Salomon D. Organotypic cultures of autologous hair follicle keratinocytes for the treatment of recurrent leg ulcers. J Am Acad Dermatol. 2003 Feb; 48(2):207-14.).
Dans ce cas spécifique, la peau reconstruite permet aussi de réaliser de multiples microautogreffes chez des patients souffrant de vitiligo de manière à repigmenter des zones déficitaires en coloration, dans des conditions meilleures que celles existantes employant de cellules de l'ORS contenant peu de mélanocytes.

Pour toutes ces applications, l'équivalent de peau selon l'invention présente l'avantage de se pigmenter naturellement. La zone cutanée greffée est ainsi plus esthétique et reproduit un comportement physiologique normal lors de l'exposition au soleil.

### Exemple I - Préparation d'un modèle de peau pigmentée

Asselineau D, Bernard B, Bailly C, Darmon M. Epidermal morphogenesis and induction of the 67 kD keratin polypeptide by culture of human keratinocytes at the liquid-air interface. Exp Cell Res. 1985 Aug; 159(2):536-9.

Asselineau D, Bernard BA, Darmon M. Three-dimensional culture of human keratinocytes on a dermal équivalent A model system to study epidermal morphogenesis and differentiation in vitro. Models dermatol, vol.3, pp. 1-7(Karger, Basel 1987)

### I-A- Préparation de l'équivalent de derme

Les fibroblastes sont décongelés, cultivés dans le milieu pour fibroblastes (MEM +10 % de FCS (sérum foetal de veau) et trypsinés.

Pour la réalisation des lattices, les cellules sont utilisées à confluence entre 12 et 13 jours après la décongélation et sont préparées de façon à obtenir une suspension cellulaire en MEM Hépes 10 % FCS à 2.106 cellules/ml.

### Préparation des milieux de culture

### MEM 1.76X

MEM 10X 17.6 ml
NaHCO3 7.5 % soit 5.1 ml
1.76 mM L-Glutamine soit 0.88 ml
0.88 mM Sodium Pyruvate soit 0.88 ml
0.88 X Acides aminés non essentiels soit 0.88 ml
8.8 U / 8.8 µg/ml (0.088 %) Penicilline Streptomycine soit 0.088 ml
0.04 X (0.04 %) antibiotique anti-mycotique soit 0.044 ml
Eau ultra pure stérile 75 ml

### MEM Hépes 10 % FCS

MEM 25 mM Hépes 50 ml
2 mM L-Glutamine soit 0.5 ml
1 mM Sodium Pyruvate soit 0.5 ml
1 X Acides aminés non essentiels soit 0.5 ml
20 U / 20 µg/ml (0.2 %) Penicilline Streptomycine soit 0.1 ml
0.1 X (0.1 %) antibiotique anti-mycotique soit 0.05 ml
10% FCS soit 5 ml

### NaOH 0.1N

10 ml NaOH 1N
90 ml eau ultra pure stérile
Cette solution est passée sur filtre Millipore à membrane GV Durapore 0.22 µm

### Acide acétique 1/1000

0.5 ml acide acétique glacial 100 %
499.5 ml eau ultra pure stérile

### Préparation des lattices

Utilisation de collagène Gattefossé
3.22 ml MEM 1.76 X
0.63 ml FCS
0.35 ml NAOH 0.1N
0.2 ml MEM Hépes 10 % FCS
Volume =4.4 ml

A cette solution ajouter 0.5 ml de suspension cellulaire.

Puis introduire lentement le volume nécessaire de collagène froid.

Vider le contenu de l'erlenmeyer dans une boite bactério Falcon ∅ 60 mm.

Placer la boîte à l'étuve (37°C - 5 % CO2) pendant environ 1 h 30 - 2 h.

Laisser la contraction se faire pendant 3 jours.

Ensemencer ensuite les cellules de la matrice.

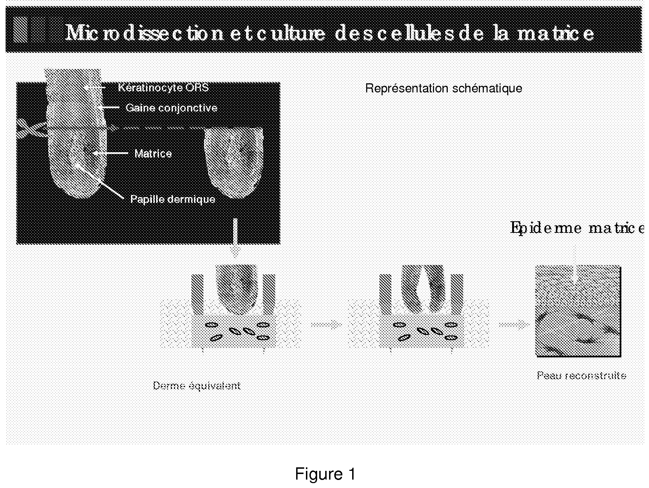

### I-B- Microdissection et mise en culture (ensemencement) des cellules de la matrice (Figure 1)

À partir d'une biopsie de peau, on réalise une microdissection des follicules pileux qu'on place dans une boîte de pétri contenant du milieu de culture. On sépare l'épithélium du bulbe de la papille dermique et de la gaine conjonctive et on coupe la région bulbaire au sommet de la papille dermique, ce fragment et ensuite placé sur le derme équivalent à l'intérieure d'un anneau stérile en exerçant une légère pression à l'aide d'une aiguille, la séparation de la gaine conjonctive avec les cellules de la matrice se fait plus facilement sans recours aux enzymes protéolytiques on élimine donc la partie dermique et on met en culture les cellules de la matrice avec le milieu de culture 3F pendant une semaine.

### I-C- Obtention de la peau reconstruite

Après 7 jours de culture en immersion, les peaux sont mises en émersion.

Vérifier toutefois que les kératinocytes sortent de la lattice en observant au microscope. Dans une boîte bactério Falcon, placer une grille d'émersion.

Ajouter 7.5 ml MEM 10 % FCS + 3 F en évitant la formation de bulles.

Découper la colle autour de la peau à émerger à l'aide d'un scalpel stérile.

Transférer la peau sur la grille avec une pince courbe et un cell-lifter.

Placer les boîtes à l'étuve 37°C-5 % CO2.

Changer le milieu le mercredi et le vendredi (7.5 ml MEM 10 % FCS + 3 F).

Après 7 jours d'émersion, les peaux sont prêtes à être utilisées.
Milieu de culture utilisé : **MEM 10 % FCS + 3 F**
MEM 500 ml
2 mM L-Glutamine soit 5 ml
1 mM Sodium Pyruvate soit 5 ml
1 X Acides aminés non essentiels soit 5 ml
20 U / 20 µg/ml (0.2 %) Penicilline Streptomycine soit 1 ml
0.1 X (0.1 %) antibiotique anti-mycotique soit 0.5 ml
10 ng/ml EGF soit 0,5 ml de la solution stock à 10 µg/ml
10-10 M Choléra Toxine soit 5 µl de la solution stock à 10-5M
0.4 µg/ml Hydrocortisone soit 0.4 ml de la solution stock à 0.5 mg/ml
10 % FCS soit 50 mi

Les figures 2 et 3 représentent des coupes transversales de peau reconstruite selon le procédé de l'invention.

Leur observation montre un épiderme stratifié surmonté d'un *stratum corneum* reproduisant un épiderme *in vivo.* Il est également visible que l'épiderme de la figure 3 comporte de la mélanine.

### Exemple II - Préparation d'un modèle de peau comportant un follicule pileux

Les conditions expérimentales des protocoles décrites dans l'exemple I seront reproduites en utilisant des fibroblastes folliculaires à l'étape I-A-.

## Revendications

1. Utilisation de cellules de la matrice, cellules localisées dans le bulbe pileux et formant un petit amas cellulaire autour de la papille dermique, pour la préparation d'un équivalent d'épiderme capable de se pigmenter.

2. Procédé de préparation d'un équivalent d'épiderme comprenant au moins une étape de mise en culture de cellules de la matrice, cellules localisées dans le bulbe pileux et formant un petit amas cellulaire autour de la papille dermique, sur un équivalent de derme.

3. Procédé selon la revendication 2, **caractérisé en ce qu'**il comprend :
a- l'ensemencement de cellules de la matrice, cellules localisées dans le bulbe pileux et formant un petit amas cellulaire autour de la papille dermique, sur un équivalent de derme reposant sur un support immergé dans un milieu de culture ;
b- la multiplication des cellules de la matrice cellules localisées dans le bulbe pileux et formant un petit amas cellulaire autour de la papille dermique, à la surface dudit équivalent de derme ;
c- l'élévation dudit support de telle sorte que le milieu de culture ne recouvre pas la face supérieure de l'équivalent d'épiderme en cours d'élaboration.

4. Procédé selon la revendication 3, **caractérisé en ce que** l'équivalent de derme de l'étape (a) est une lattice de collagène dans laquelle se répartissent des fibroblastes dermiques.

5. Procédé selon la revendication 4, caractérisé en que lesdits fibroblastes sont des fibroblastes interfolliculaires.

6. Procédé selon l'une quelconque des revendications 3 à 5, **caractérisé en ce que** l'étape b consiste à maintenir les cellules de la matrice en contact avec l'équivalent de derme immergé dans un milieu nutritif pendant 5 à 6 jours.

7. Procédé selon l'une quelconque des revendications précédente, **caractérisé en ce que** le milieu nutritif est un milieu 3F.

8. Procédé selon l'une quelconque des revendications précédentes, **caractérisé en ce qu'**il comprend en outre une étape induisant la pigmentation.

9. Equivalent d'épiderme susceptible d'être obtenu par le procédé selon l'une quelconque des revendications 2 à 8, comportant des mélanocytes avec trois dendrites ou plus et constitué de cellules issues de la prolifération et de la différentiation de cellules de la matrice, comprenant au moins des kératinocytes et des mélanocytes

10. Equivalent d'épiderme selon à la revendication 9, comprenant en outre des cellules de Langherans et/ou de Merkel.

11. Equivalent de peau **caractérisé en ce qu'**il comprend un équivalent d'épiderme selon l'une quelconque des revendications 9 et 10.

12. Utilisation d'un équivalent de peau selon la revendication 11 pour tester la libération et/ou la pénétration et/ou l'absorption et/ou la biodisponibilité cutanée et/ou la tolérance et/ou la compatibilité et/ou l'efficacité de composés.

13. Utilisation selon la revendication 12 dans des procédés automatisés de criblages de composés cosmétiques, pharmaceutiques ou dermatologiques.

14. Utilisation selon la revendication 12 ou 13 pour l'identification de composé modulateur de la pigmentation de la peau.

15. Utilisation selon la revendication 12 ou 13 pour l'identification de composé modulateur de la pousse des poils et/ou des cheveux

16. Utilisation selon la revendication 12 ou 13 pour l'identification de composé susceptibles d'induire des réactions cutanées d'irritation ou d'allergie.

17. Procédé de criblage de produit en vue d'identifier de nouveaux actifs comprenant une étape (a) de mise en contact dudit produit à tester avec un épiderme équivalent selon l'une quelconque des revendications 9 et 10 puis une étape (b) de lecture de l'effet dudit produit sur l'équivalent d'épiderme.

18. Utilisation d'un équivalent d'épiderme selon l'une quelconque des revendications 9 et 10 pour la préparation de greffons destinés à traiter des désordres cutanés choisis parmi une brûlure, un défaut de cicatrisation, un ulcère cutané, le psoriasis, un désordre pigmentaire tel que le vitiligo, l'hypomélanoses ou l'hypermélanoses congénitales ou acquises ou dû à une cause chirurgicale.

## Patentansprüche

1. Verwendung von Matrixzellen, in der Haarzwiebel befindlichen Zellen, die einen kleinen Zellhaufen um die Dermispapille herum bilden, zur Herstellung eines Epidermis-Äquivalents, das zur Pigmentierung fähig ist.

2. Verfahren zur Herstellung eines Epidermis-Äquivalents, das mindestens einen Schritt zum Kultivieren von Matrixzellen, in der Haarzwiebel befindlichen Zellen, die einen kleinen Zellhaufen um die Dermispapille herum bilden, auf einem Dermis-Äquivalent umfasst.

3. Verfahren nach Anspruch 2, **dadurch gekennzeichnet, dass** es Folgendes umfasst:
a - Beimpfen eines Dermis-Äquivalents, das auf einem Träger liegt, der in einem Kulturmedium eingetaucht ist, mit Matrixzellen, in der Haarzwiebel befindlichen Zellen, die einen kleinen Zellhaufen um die Dermispapille herum bilden;
b - Vermehren der Matrixzellen, in der Haarzwiebel befindlicher Zellen, die einen kleinen Zellhaufen um die Dermispapille herum bilden, an der Oberfläche des Dermis-Äquivalents;
c - Anheben des Trägers derart, dass das Kulturmedium während der Herstellung nicht die Oberseite des Epidermis-Äquivalents bedeckt.

4. Verfahren nach Anspruch 3, **dadurch gekennzeichnet, dass** das Dermis-Äquivalent aus Schritt (a) ein Kollagen-Gitter ist, in dem Fibroblasten der Dermis verteilt sind.

5. Verfahren nach Anspruch 4, **dadurch gekennzeichnet, dass** die Fibroblasten interfollikuläre Fibroblasten sind.

6. Verfahren nach einem der Ansprüche 3 bis 5, **dadurch gekennzeichnet, dass** Schritt b darin besteht, die Matrixzellen mit dem Dermis-Äquivalent in Kontakt zu halten, das 5 bis 6 Tage lang in einem Nährmedium eingetaucht ist.

7. Verfahren nach einem der vorhergehenden Ansprüche, **dadurch gekennzeichnet, dass** das Nährmedium ein 3F-Medium ist.

8. Verfahren nach einem der vorhergehenden Ansprüche, **dadurch gekennzeichnet, dass** es ferner einen Schritt umfasst, mit dem die Pigmentierung veranlasst wird.

9. Epidermis-Äquivalent, das sich mit dem Verfahren nach einem der Ansprüche 2 bis 8 erhalten lässt und Melanozyten mit drei oder mehr Dendriten aufweist und aus Zellen gebildet ist, die von der Proliferation und der Differenzierung von Matrixzellen stammen, und das mindestens Keratinozyten und Melanozyten umfasst.

10. Epidermis-Äquivalent nach Anspruch 9, das ferner Langerhans- und/oder Merkel-Zellen umfasst.

11. Hautäquivalent, **dadurch gekennzeichnet, dass** es ein Epidermis-Äquivalent nach einem der Ansprüche 9 und 10 umfasst.

12. Verwendung eines Hautäquivalents nach Anspruch 11 zum Testen der Abgabe und/oder Eindringung und/oder Adsorption und/oder der dermalen Bioverfügbarkeit und/oder Verträglichkeit und/oder Kompatibilität und/oder Wirksamkeit von Verbindungen.

13. Verwendung nach Anspruch 12 in automatisierten Verfahren zum Screening von kosmetischen, pharmazeutischen oder dermatologischen Verbindungen.

14. Verwendung nach Anspruch 12 oder 13 zum Ermitteln einer die Hautpigmentierung beeinflussenden Verbindung.

15. Verwendung nach Anspruch 12 oder 13 zum Ermitteln einer das Wachstum von Haut- und/oder Körperhaaren beeinflussenden Verbindung.

16. Verwendung nach Anspruch 12 oder 13 zum Ermitteln einer Verbindung, die Hautreizungen oder allergische Hautreaktionen bewirken kann.

17. Verfahren zum Produkt-Screening zwecks Ermittlung neuer Wirkstoffe, das einen Schritt (a) zum In-Kontakt-Bringen des zu testenden Produkts mit einem Epidermis-Äquivalent nach einem der Ansprüche 9 und 10 und anschließend einen Schritt (b) zum Bestimmen der Winkung des Produkts auf das Epidermis-Äquivalent umfasst.

18. Verwendung eines Epidermis-Äquivalents nach einem der Ansprüche 9 und 10 zur Herstellung von Transplantaten zum Behandeln von Hautstörungen, ausgewählt aus einer Verbrennung, einer Wundheilungsstörung, einem Geschwür der Haut, Schuppenflechte, einer Pigmentstörung wie Vitiligo, angeborenen oder erworbenen Hypomelanosen oder Hypermelanosen oder verursacht durch eine Operation.

## Claims

1. Use of matrix cells, which cells are located in the hair bulb and form a small cell cluster around the dermal papilla, for the preparation of an epidermis equivalent capable of pigmenting.

2. Method of preparing an epidermis equivalent comprising at least one step for culturing matrix cells, which cells are located in the hair bulb and form a small cell cluster around the dermal papilla, on a dermis equivalent.

3. Method according to Claim 2, **characterized in that** it comprises:
a- the inoculation of matrix cells, which cells are located in the hair bulb and form a small cell cluster around the dermal papilla, on a dermis equivalent resting on a support immersed in a culture medium;
b- the multiplication of the matrix cells, which cells are located in the hair bulb and form a small cell cluster around the dermal papilla, at the surface of the said dermis equivalent;
c- the elevation of the said support such that the culture medium does not cover the top face of the epidermis equivalent during production.

4. Method according to Claim 3, **characterized in that** the dermis equivalent of step (a) is a collagen lattice in which dermal fibroblasts are distributed.

5. Method according to Claim 4, **characterized in that** the said fibroblasts are interfollicular fibroblasts.

6. Method according to any one of Claims 3 to 5, **characterized in that** step b consists in maintaining the matrix cells in contact with the dermis equivalent immersed in a nutrient medium for 5 to 6 days.

7. Method according to any one of the preceding claims, **characterized in that** the nutrient medium is a 3F medium.

8. Method according to any one of the preceding claims, **characterized in that** it additionally comprises a pigmentation-inducing step.

9. Epidermis equivalent capable of being obtained by the method according to any one of Claims 2 to 8, comprising melanocytes with three or more dendrites and consisting of cells derived from the proliferation and the differentiation of matrix cells comprising at least keratinocytes and melanocytes.

10. Epidermis equivalent according to Claim 9, additionally comprising Langerhans and/or Merkel cells.

11. Skin equivalent, **characterized in that** it comprises an epidermis equivalent according to either of Claims 9 and 10.

12. Use of a skin equivalent according to Claim 11, for testing skin release and/or penetration and/or absorption and/or bioavailability, and/or the tolerance and/or compatibility and/or efficacy of compounds.

13. Use according to Claim 12, in automated methods of screening cosmetic, pharmaceutical or dermatological compounds.

14. Use according to Claim 12 or 13, for the identification of a compound modulating skin pigmentation.

15. Use according to Claim 12 or 13, for the identification of a compound modulating the growth of body hair and/or head hair.

16. Use according to Claim 12 or 13, for the identification of a compound capable of inducing skin irritation or allergy reactions.

17. Method of screening a product in order to identify novel active agents, comprising a step (a) for bringing the said test product into contact with an epidermis equivalent according to either of Claims 9 and 10 and then a step (b) for reading the effect of the said product on the epidermis equivalent.

18. Use of an epidermis equivalent according to either of Claims 9 and 10, for the preparation of grafts intended to treat skin disorders chosen from a burn, a wound healing defect, a skin ulcer, psoriasis, a pigmentation disorder such as vitiligo, congenital or acquired hypomelanosis or hypermelanosis, or due to a surgical cause.
